Europäisches Patentamt

European Patent Office

Office européen des brevets

(19)

(11) Publication number: **0 446 931 A1**

# EUROPEAN PATENT APPLICATION

(21) Application number: **91103969.1**

(22) Date of filing: **14.03.91**

(51) Int. Cl.5: **C12N 15/12**, C07H 21/04,
C12N 1/21, C12N 5/10,
C12N 5/16, C07K 13/00,
A61K 37/02

| | |
|---|---|
| The applicant has subsequently filed a sequence listing and declared, that it includes no new matter. | (71) Applicant: **FIDIA S.p.A.**<br>**Via Ponte della Fabbrica 3-A**<br>**I-35031 Abano Terme (Padova)(IT)** |
| (30) Priority: **14.03.90 IT 4155790**<br>**17.07.90 IT 4165590** | (72) Inventor: **Della Valle, Francesco**<br>**Via Cerato 14**<br>**Padova(IT)** |
| (43) Date of publication of application:<br>**18.09.91 Bulletin 91/38** | Inventor: **Callegaro, Lanfranco**<br>**Via Bravi, 35**<br>**Padova(IT)** |
| (84) Designated Contracting States:<br>**AT BE CH DE DK ES FR GB GR IT LI LU NL SE** | Inventor: **Negro, Alessandro**<br>**Via Umago 5**<br>**Padova(IT)** |
| | (74) Representative: **Vossius & Partner**<br>**Siebertstrasse 4 P.O. Box 86 07 67**<br>**W-8000 München 86(DE)** |

(54) **Human ciliary neuronotrophic factor, DNA sequence encoding the factor, and production of the factor by recombinant technology.**

(57) The present invention relates to human ciliary neuronotrophic factor and, in particular, the discovery of the gene which encodes the factor, the amino acid sequence of the factor and methods of producing the same by means of recombinant DNA technology.

EP 0 446 931 A1

Rank Xerox (UK) Business Services

The present invention relates to human ciliary neuronotrophic factor and, in particular, the discovery of the gene sequence segment of DNA which encodes the human ciliary neuronotrophic factor, the amino acid sequence of the factor and methods of producing the same by means of recombinant DNA technology. In this way it is possible to obtain significant quantities of homogeneous protein with important biological activities, and free from contaminating proteins of human origin.

The ciliary ganglion (CG) contains two populations of neurons, ciliary and choroid, both of which are cholinergic. CG neurons innervate the intrinsic muscle structures of the eye in the choroid, the ciliary body and the iris. In chick embryo, during development, about half of the CG neurons die between days E8 and E15, when their axons establish a connection with the intraocular innervation territory.

This neuronal death is strongly favored by removal of the eye, while it is significantly impeded by the implantation of a supplementary eye bud. On the basis of this observation it has been hypothesized that the innervation territories contain trophic factors for their own specific neurons. Extracts from various chick embryo tissues at day E8 have proved to have trophic activity on neurons dissociated from the ciliary ganglia of chick embryos at the same stage of development (Adler 1979).

This trophic activity was subsequently named ciliary neuronotrophic factor (CNTF) (Barbin 1982).

Neurons dissociated from chick embryo ciliary ganglia at day E8, seeded on a suitable substrate and with a suitable culture medium, do not survive unless a special supplement is added to the medium, making a model for biological tests for ciliary neuronotrophic factors in general, and in particular for those of a proteic nature. When various extracts of chick embryo tissue at day E8 are compared in this regard, a third of the total CNTF activity of the embryo is found in the eye, and three quarters of the activity of the eye itself is localized in a portion of the choroid, the iris and the ciliary body (intrinsic muscle structure of the eye) as well as the pigmented epithelium (CIEP). Assessments of survival showed that a soluble protein, trophically active for CG neurons, (i) is found at high concentrations in the eye territory which they innervate, and (ii) reaches its highest specific activity between E8-E15, the very developmental stage at which the fate of these neurons is decided in vivo. The purification of E15 chick embryonic CNTF involves the selective subdissection of CIEP tissues and also sequential treatment of the relative extract by ion exchange chromatography, ultracentrifugation in sucrose gradient, and sodium dodecyl sulfate polyacrilamide gel electrophoresis (SDS-PAGE).

Purification of this protein by ion exchange chromatography, ultracentrifugation in a sucrose gradient and SDS-PAGE under reducing conditions revealed a molecular weight of approximately 21kd and a net negative charge. The trophic activity exercised by the purified protein on chick CG neurons at stage E8, on chick DRG neurons at stage E10 and on sympathetic neurons at stage E11 is in the order of $10^{-11}$ $10^{-12}$M and therefore of the same order as another neuronotrophic factor, namely Nerve Growth Factor (NGF), purified from submaxillary glands of male mice, with regard to its DRG and sympathetic neuronal targets.

Chick embryo eye extract, like the CIEP extract, has a trophic activity (i) on E10 (but not E8) chick embryonic DRG neurons and newborn mouse neurons, and (ii) on sympathetic neurons from E11 chick and newborn rat ganglia - not surprisingly, considering that the innervation territories of the sensitive and sympathetic neurons are partly the same as those of the CG neurons. DRG and sympathetic neurons on the other hand are sensitive to the trophic effect of purified chick eye CNTF, despite the fact that CNTF was chosen for its trophic activity on CG neurons.

Like NGF, CNTF supports the growth of adrenal chromaffin cells in culture although these two factors differ in the extent of their effects on hydroxylase tyrosine and phenylethanolamine N-methyltransferase enzymes. CNTF enhances CAT activity in chick retinal cell cultures, indicating a response of cholinergic neurons. CNTF has no trophic effects on cholinergic neurons of the rat embryonic basal forebrain or pontine region of the brain and cannot be considered a cholinergic neuronotrophic factor. Very recent data indicate that the CNTF's action is not restricted to neuronal cells. CNTF was found to induce the differentiation of glial progenitor cells of the rat optic nerve into type-2 astrocytes.

Among CNTF's other activities is one linked with the prevention of motoneuron degeneration following axotomy (Sendtner 1990).

To summarize, this new factor is represented by a protein which is active on at least three types of neuron, two of which are also sensitive to NGF. Bearing in mind its molecular weight, chick eye CNTF promotes half the maximum survival of CG neurons of E8 chick, DRG neurons of E10 chick and sympathetic neurons of E11 chick at a concentration of $10^{-11}$--$10^{-12}$M, with a specific activity of the same order as that of purified NGF from mouse submaxillary gland on its neuronal targets, DRG and sympathetic. None of the trophic activities of check eye CNTF is blocked or inhibited by antibodies directed against the beta subunit of NGF from mouse submaxillary gland.

Trophic factors for CG neurons, as for DRG and sympathetic neurons, have been obtained from various other sources, ranging from tissue extracts and lesion fluids to media conditioned by various cell cultures.

"Conditioned culture media" have an advantage over tissue extracts, namely that of facilitating the identification of "cell sources". On the other hand, due to the low concentrations of trophic activity which can be obtained from them, these conditioned media are not very suitable for the preparation of purified proteins with trophic activity. Indeed, conditioned media from heart muscle cultures were the first materials (closely followed by skeletal muscle cultures) in which the presence of CNTF was demonstrated. Bovine heart extracts have also been considered as a possible starting material, but the complete characterization of their CNTF has proven impossible. The presence of a CNTF in the heart and skeletal muscle leads to the assumption that it may be active on viscero- and somato-motor cholinergic neurons other than those (CG) which innervate the intrinsic muscles of the eye. However, a systematic comparison between CNTF of the eye, heart and skeletal muscles has not yet been carried out, either with regard to their molecular characteristics or range of neuronal targets.

Conditioned media from neuroglial cell cultures, both peripheral (Schwann) and central (astroglia), also show trophic activities, different from those of NGF, on ciliary neurons and those of the dorsal and sympathetic root ganglia, decidedly favoring the hypothesis of a possible role of the neuroglia as a source of neuronotrophic factors in vivo.

It has also been demonstrated that extracts from adult rat sciatic nerve, unlike those of CNS tissues, have a very high CNTF content, with a specific activity equal to that of CIEP from E15 chick eye (about 20,000 trophic units/mg of protein). CNTF's activity is also high in extracts from nerves or from pure motor roots or pure sensory roots, demonstrating that no differential criterion can justify correlating the activity of the nerve with the specific innervation territories, rather than with the Schwann cells contained within its structure, or together with them.

Manthorpe and co-workers (Manthorpe et al., Ciliary Neuronotrohic Factors in Nerve Growth Factors, R.A. Rush Ed., Ibro Handbook Series: Methods in the Neurosciences, Vol. 12, pp. 31-56) purified rat nerve CNTF at an analytical level, revealing a slightly inferior negative charge and a slightly higher molecular weight (24kD) than those of CNTF purified from chick eye.

CNTF has now also been isolated from rat sciatic nerve, bovine heart and neuroblastoma cells. Williams et al. demonstrated a high CNTF content in the sciatic nerve of adult rats, with a specific activity equal to that of chick (20,000 TU/mg protein).

Two forms of CNTF have been identified, one with a molecular weight of about 25 Kd and another of about 28 Kd. By homogenous purification of quantities of CNTF from rabbit and rat sciatic nerve it has been possible to partially identify its amino acid sequence (Lin L-F, H., J. Bio. Chem., Vol. 265, No. 15, pp. 8942-8947, 1990). With this knowledge and by using the PCR (Polymerase Chain Reaction) technique the complete gene segment encoding the CNTF of these two animals was isolated (Stöckli et al, Nature, Vol. 342, pp. 920-923, 1989, Lin L-F.H. et al, Science, Research Articles, Vol. 246, pp. 1023-1025, 1989). The molecular weight of the protein was 22.7 and 22.8 Kd respectively, with an isoelectric point of 5.78 in the first case, as for the purified proteins.

Comparative analysis of the CNTF sequences of rat and rabbit has shown them to be highly homologous both in amino acid and nucleotide sequences. Some of the better preserved domains of this molecule have been successfully determined by computer analysis, allowing the identification and isolation of a significant portion of the human gene segment, corresponding to 80% of the complete sequence and using the PCR technique some oligonucleotides were synthesized in these very domains. This portion of gene sequence and the corresponding amino acid sequence are described below.

Recombinant DNA technology makes it possible to construct series of vectors able to express large quantities of proteins and allows molecular biologists to assemble DNA sequences to create hybrid molecules capable of producing the protein of interest. Various reactions are used, such as cutting with restriction enzymes, joining the fragments thus obtained with ligase, the chemical synthesis of oligonucleotides to be assembled and other methodologies devised at various laboratories working in this field (Maniatis, T. et al, Molecular Cloning. A Laboratory Manual. Cold Spring Harbor Laboratory, Cold Spring Laboratory NY, 1982).

To obtain high levels of expression, the DNA elements to be assembled must present certain essential information. For example, a replication origin, a selection for antibiotics, an expression promoter, activators of the transcription of the gene of interest and other characteristics known to the expert in the art. Suitable combination of these elements results in a plasmid, if the gene of interest is inserted naturally with respect to the regulatory sequences of the transcription and translation and the resulting plasmid is called an expression plasmid. The expression plasmid or vector is thus able to express the protein in host cells, which may be of eukaryotic or prokaryotic origin. The protein can therefore be obtained by purification.

The elements (promoters) which naturally control the expression of many genes such as growth factors, are not strong in their expression and are activated only in suitable natural conditions which are often unknown. To this end, promoters with known activity are used, such as viruses of the Papovavirus series, or other known promoting gene sequences. The elements used for high levels of expression are therefore a combination of DNA of various origin (eukaryotic, bacterial, viral, etc.) constituted in the terminal part by different gene portions bound together to form a hybrid.

The transcription activity of a gene depends on the distances between the regulatory and encoding sequences.

Given these facts, one of the best ways for the regulatory sequences to work suitably is that the introduced gene be placed in the same position as the natural gene. One system used requires the regulatory sequences to include some amino acids of the encoding sequences. Union with the introduced gene gives rise to a fusion protein which is then removed, obtaining higher biological values. Should the fusion protein technique not be used, conventional technology to obtain genes situated in the close vicinity of regulatory sequences depends on the existence of suitable restriction sites to allow their cloning. If there are no compatible sites in the vicinity, but only other sites, it is possible to obtain a union of the segments by synthesis of an oligonucleotide or linker containing the desired restriction site.

If there are no suitable restriction sites in the vicinity so that a linker would be of no use, then the technique of deletion of the DNA with Bal31 or SI is used. This does not allow a precise deletion however and it is necessary to check each time by sequencing the various clones to see which is most suitable.

Even though the techniques used to isolate proteins with new biological activities conventionally involve isolating the protein itself by purification from biological fluids, the quantity of protein which can be obtained is not always sufficient to allow it to be used subsequently to study its structure, functions and, above all, applications. Recombinant DNA technology can be used to obtain appreciable quantities of protein with which to clone the protein itself in an expression vector. Sometimes, however, as in the case of the present invention, nothing is known of the primary structure of the protein and conventional cloning is not possible. In the case of CNTF, the only worthwhile information known before the present invention was that rabbit and rat genes have been isolated and their relative nucleotide sequences determined (K.A. Stöckli et al., Nature, vol. 342, 1989, page. 920-923; Leu-Fen H. Lin et al., Science, vol. 246, 1989, page 1023-1025).

On this basis, it might be possible to isolate human CNTF in the conventional way using one of these two very genes as probe and to screen a bank of human genes from cDNA or genomic DNA.

However, these systems are very limiting for the molecular biologist and consequently alternative strategies must be developed in view of the new techniques now available, such as the Polymerase Chain Reaction (PCR) (Saiki et al, Science 239:487, 1988; Scharf, S.J., Science 233: 1076, 1986). By this technique it is possible to amplify a gene segment up to $10^6$ times. It involves the use of two oligonucleotides each of which can hybridize to alternative strands of a DNA to be amplified. The distance between the two oligonucleotides with respect to the gene sequence gives the dimensions of the molecule to be produced. These two oligonucleotides are so constructed that there is a restriction site within their sequence which allows their subsequent cloning. This restriction site is either naturally present or is constructed ad hoc by degenerating the minimum number of nucleotides.

This approach, which can be defined as site-directed mutagenesis, allows restriction sites to be constructed in positions previously decided on by the molecular biologist. The construction of sites compatible with other gene segments not only facilitates cloning but also makes it possible to specifically join different gene segments. A series of oligonucleotides has been constructed with sequences based on the knowledge of rabbit and rat genes.

This technique can be described as cloning by direct mutagenesis. Thanks to recombinant DNA technology it is possible in practice to express complete heterologous polypeptides by direct expression or alternatively the heterologous polypeptide can be expressed, fused to a portion of an amino acid sequence of an analogous polypeptide. In general, products thus obtained are not biologically active (British Patent Application Publ. N. 2007676A; Wenzel, American Scientist 68, 664, 1980).

CNTF is one of many growth factors currently being studied to identify the specificity of their biological activity and to devise a method of obtaining them by recombinant DNA technology or other purification and extraction techniques. Such growth factors, including CNTF, can be used therapeutically for maintenance or preventive purposes, recovery of nervous function, in acute or chronic pathological conditions including those of a neurodegenerative or immunoneurodegenerative nature affecting the peripheral, central and vegetative nervous systems.

However, there still are serious limitations for said therapeutical uses of CNTF, because this protein is only available in insufficient amounts. The technical problem underlying the present invention therefore is to provide DNA-sequences encoding CNTF, vectors for the expression of CNTF, microorganisms and cell lines capable of expression of CNTF, CNTF, optionally in substantially pure form, and pharmaceutical compositions containing said CNTF, the protein and the pharmaceutical composition which may be used for the treatment of nervous disorders or the maintenance, prevention of loss or recovery of nerve function. The solution to this problem is provided by the embodiments characterized in the claims.

The present invention, therefore, relates to the human gene which encodes human CNTF, to methods for the isolation and sequencing of said gene, the identification of the corresponding amino acid sequence of human CNTF, and the production of commercially useful and biologically active amounts of the protein by recombinant DNA technology, free from contaminating proteins of human origin.

The present invention also relates to DNA sequences that hybridise to the above-mentioned DNA sequence and that encode a polypeptide having the biological activity of CNTF. In this context, the term "hybridisation" preferably refers to conventional hybridisation conditions under which the $T_m$ value is between $T_m$-20 to $T_m$-27. In particularly preferred embodiments, the term "hybridisation" refers to stringent hybridisation conditions.

The invention also relates to pharmaceutical compositions comprised of human CNTF, either alone or in combination with gangliosides (including naturally existing gangliosides, ganglioside derivatives, and semisynthetic analogues of gangliosides), polysaccharides (including natural and chemically modified polysaccharides), and/or other growth factors.

The invention further relates to the therapeutic use of human CNTF, and the above noted pharmaceutical compositions.

The figures show:

Figure 1: partial sequence of the gene encoding human CNTF.

Figure 2: translation of the Figure 1 sequence into the corresponding amino acids with the three possible reading frames.

Figure 3 : amino acid sequence coded for by the nucleotide sequence of Figure 1.

Figure 4: comparison of the amino acid sequence shown in Figure 3 with the rat and rabbit CNTF sequences.

Figure 5: comparison of the hydropathicity profiles of the amino acid sequence shown in Figure 3 with that of the rat and rabbit CNTF.

Figure 6: nucleotide sequence of the human CNTF gene from human genome libraries in EMBL-3.

Figure 7: nucleotide sequence of the human CNTF gene from a human retina library in gt11.

Figure 8: amino acid sequence of the human CNTF encoded by the nucleotide sequence shown in Figure 7.

Figure 9: construction of the plasmid pSVCNTFh for the prokaryotic expression of human CNTF.

Figure 10: construction of the plasmid pJLACNTF for the eukaryotic expression of human CNTF.

Figure 11: graph of the biological activity assessment results for CHO cells transfected with the gene for human CNTF.

In investigating the sequence of the human CNTF gene, the present inventors first succeeded in sequencing a portion of the gene and then, utilizing this knowledge successfully sequenced the entire gene, leading to an identification of the amino acid sequence of human CNTF.

The procedures utilized and described herein include various general procedures which are applicable to the specific steps set forth herein below.

Before explaining in detail the experiments that have been carried out it seems expedient to describe the materials and general procedures used therein.

Materials and Methods

Some reactions and procedures are well known to those skilled in the art and are described, for example, in Molecular Cloning, Sambrook J. et al. (1989) and Mantiatis, T. et al., Molecular Cloning, A Laboratory Manual, Cold Spring Harbor Laboratory, Cold Spring Laboratory, N.Y. (1982).

Cutting and attachment of the DNA segments with restriction enzymes is done according to the manufacturer's instructions. Generally, 1 $\mu$g of plasmid was cut with 1 U of enzyme in 20 $\mu$l of solution; incubation temperature and time are dependent on which enzyme is used, but are generally 1 hour at 37° C. After incubation, the plasmids and gene segments are purified in all cases in an Agarose gel, LMP Agarose (BRL - Unites States of America) in 40 mM Tris HCl, 20 mM sodium acetate, 1 mM EDTA and then eluted from the agarose with a GENECLEANTM kit (BIO 101 Inc, La Jolla, CA - USA).

Ligation is performed with T4 ligase at a concentration of 1 U per 0.5 $\mu$g of DNA in a reaction volume of 20 $\mu$l at 13° C for 12 hours. Analyses to confirm the correct plasmid sequence are effected by transformation of HB101 cells and the transformed cells are selected in agarose plates in LB (Luria Bertani) medium with 50 $\mu$g/ml of the antibiotic ampicillin. The plasmids contained in the HB101 cells are grown in LB, 100 $\mu$g/ml of ampicillin, and are purified, for both small and large preparations, with a kit supplied by Quiagen (DIAGEN GmbH, Düsseldorf - Germany). The cloning vectors are prepared from bacterial cells according to the instructions from Quiagen.

The DNA for PCR reactions is prepared from human placenta at term as follows. A 0.4 cm³ piece of chorionic villi is finely chopped with scissors and suspended in 700 µl of 50 mM Tris/HCl pH 7.8, 100 mM EDTA, 100 mM NaCl, 1% SDS. To this is then added 35 µl of proteinase K(100 µg/ml) and the whole is incubated overnight at 55°C. 20 µl of a 13 µg/ml solution of RNAase A is then added and incubated for another 2 hours. This is followed by two extractions with phenol and two with chloroform. The DNA is then precipitated onto a fine glass tube by the addition of 1 volume of isopropanol. At this point it undergoes several passages with 70% and 100% ethanol and is then dried. The DNA is dissolved in buffer (10 mM Tris/HCl pH 7.4, 1 mM EDTA), under gentle stirring. A few hours later the dissolved DNA is ready for gene amplification. 0.1 µg of DNA normally proves sufficient for PCR.

The DNA for Southern blot is prepared as described hereafter from human placenta at term. A 0.4 cm³ piece of chorionic villi is finely chopped with scissors and suspended in 700 µl of 50 mM Tris/HCl pH 7.8, 100 mM EDTA, 100 mM NaCl, 1% SDS. To this are then added 35 µl of proteinase K(100 µg/ml) and the whole is incubated overnight at 55°C. To this are then added 20 µl of a 13 µg/ml solution of RNAase A and it is incubated for another 2 hours. This is followed by two extractions with phenol and two with chloroform. The DNA is then precipitated onto a fine glass tube by the addition of 1 volume of isopropanol. At thin point it undergoes several passages with 70% and 100% ethanol% and is then dried.

The DNA is dissolved in buffer (10 mM Tris/HCl pH 7.4, 1 mM EDTA), under gentle stirring. It is then digested with restriction enzymes at 37°C overnight. The fragments are then separated in a 0.8% Agarose gel in TAE (Tris Acetate EDTA) -buffer. The gel is washed twice in 0.1 M HCl and 1.5 M NaCl, twice in 1M NaOH 1.5M NaCl and twice in 2M Tris pH 7.4, 0.6 M NaCl, then transferred overnight to nitrocellulose (Hybond N, Amersham). The nitrocellulose filter is exposed to ultraviolet rays for 3 minutes then prehybridized in 50% Formamide, 5 x Denhardt's, 5XSSC, 1mg/ml of DNA from salmon sperm and incubated at 42°C for 2 hours. Hybridization is effected overnight in 50% Formamide, 5 x Denhardt's, 5 x SSC, 0.250 µg/ml of DNA from salmon sperm and with 10% Dextran Sodium Sulfate. The filter is washed in 0.1% SDS 2 x SSC for 2 hours at 65°C and then auto-radiographed.

All oligonucleotides are synthesized in solid phase using a 3808 DNA Synthesizer (Applied Biosystems - USA) according to the manufacturer's instructions. They are treated at 55°C for 12 hours in NH₃ and then dried in a vacuum-speed centrifuge. They are resuspended in 2.5 M ammonium acetate and then precipitated with 3 volumes of cold ethanol (-20°C). They are rewashed with cold 80% ethanol and resuspended in water. The concentration of oligonucleotides is assessed by spectrophotometry.

Amplification was effected on a Perkin Elmer Cetus DNA Thermal Cycler and the reagents used for amplification were those of the DNA™ Amplifier (Perkin Elmer-Cetus).

The examples illustrate the invention.

Example 1: Partial Sequencing of the Human CNTF Gene

To clone the human CNTF segment in a plasmid, the information provided by the rabbit CNTF nucleotide sequence obtainable through GENEBANK (access number M29828) is utilized. From this sequence two oligonucleotides are synthesized.

The first, between bases 123 and 147, contains the following sequence:
5'CAGGGCCTGAACAAGAACATCAAC3'
The eighth base is mutated to create an ApaI site and to facilitate subsequent cloning:

ApaI

5'CAGGGCCCGAACAAGAACATCAAC3'

This oligonucleotide is named ApaI.
The second oligonucleotide, including the bases between positions 581 and 600, is complementary to this sequence to allow PCR and has the following sequence:
5'CTACATTTCCTTGACGTTAG3'
On the 5' side, an SalI restriction site is added to facilitate subsequent cloning. Therefore, the following sequence is synthesized:

SalI

5'TAGTGCACTACATTTCCTTGACGTTAG3'

The two oligonucleotides are synthesized in solid phase using a 330B DNA Synthesizer (Applied Biosystems - USA) according to the manufacturer's instructions. They are treated at 55°C for 12 hours in $NH_3$ then dried in a vacuum-speed centrifuge. They are resuspended in ammonium acetate 2.5 M and precipitated with 3 volumes of cold ethanol (-20°C). They are rewashed with cold 80% ethanol and resuspended in water. The concentration of the two oligonucleotides is assessed by spectrophotometry.

Amplification is effected on a Perkin Elmer Cetus DNA Thermal Cycler and the reagents used for amplification are those of the DNA™ Amplifier kit (Perkin Elmer-Cetus). In summary, a mixture containing 200 µM of each oligonucleotide is used, 0.5 µM of each of the nucleotides dATP, dTTP, dCTP, dGTP and 0.1 µg of human DNA and reaction buffer in a total mixture of 100 µl with 0.5 U of Taq polymerase, the whole being then covered with liquid paraffin to prevent evaporation. The amplification reaction is conducted at a setting of 35 cycles in the case of human DNA and at 25 cycles in the case of purified phage DNA. The cycle in both cases is as follows: 1 min. at 94°C, 2 min. at 45°C, 3 min. at 72°C. This is slightly modified *as appropriate when the sequence to be amplified is longer.

The amplified fragment of 482 bp is purified in an agarose gel (NuSieve) Low Melting using a GENE-CLEAN™ kit (BIO 101 Inc, La Jolla, CA - USA) to dissolve the agarose. The DNA is cut with restriction enzymes Apal and Sall and repurified as above. Thus purified, the fragments are cloned using the Apal and Xhol sites of the vector pGEM-7Zf( + ) (Promega).

The amplified segment is sequenced with pUC/M13 primers which map in this plasmid between positions 2941-2957 and 158-174.

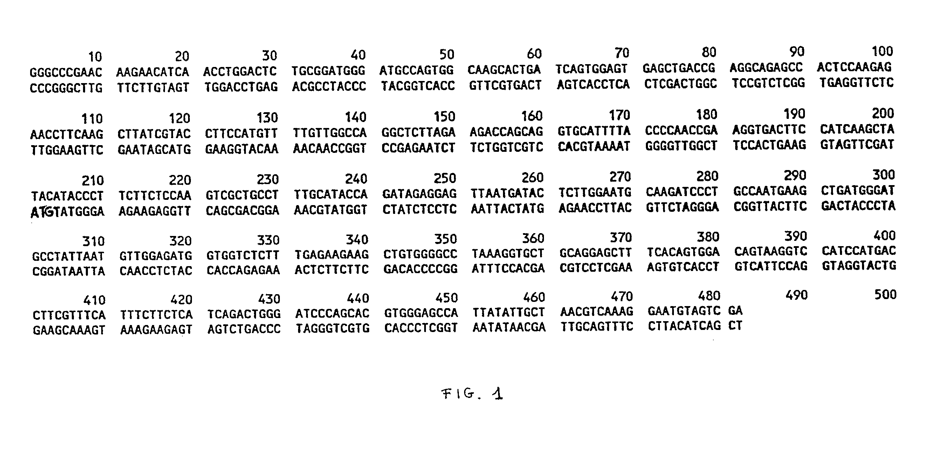

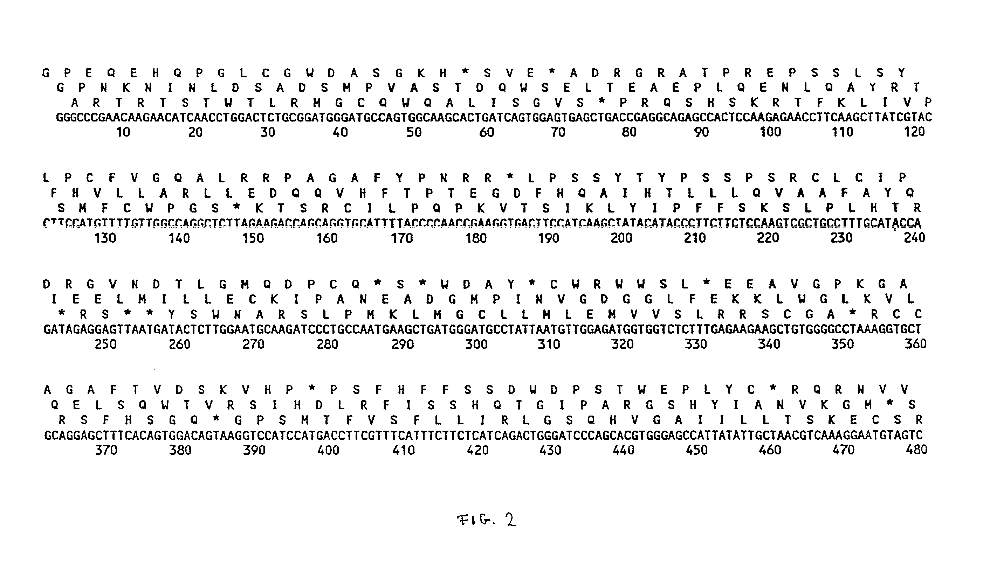

Sequenced in both directions, the sequence is that described in Figure 1 wherein A represents a deoxyadenylic acid residue, G a deoxyguanylic acid residue, C a deoxycytidylic acid residue and T a thymidylic acid residue, and the left and right ends of the Figure represent the 5'-terminus and 3'-terminus, respectively.

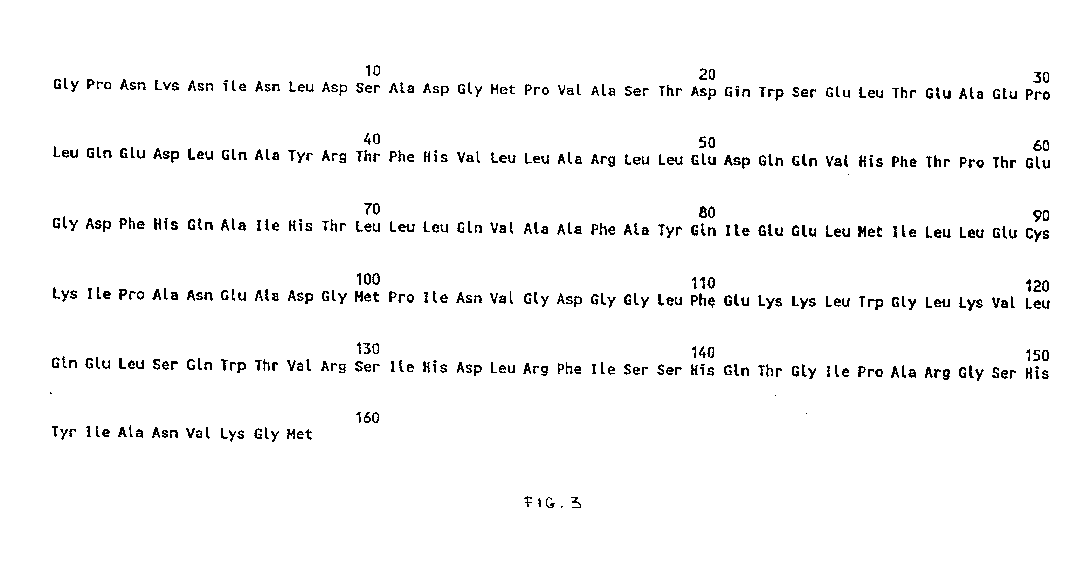

The nucleotide sequence of Figure 1 is translated into the corresponding amino acids using the three different possible reading frames. This translation is set forth in Figure 2.

As can be seen in Figure 2, translation of the second reading frame in line 2 gives a continuous gene with no stop codon. The continuous protein sequence thus defined is set forth in Figure 3.

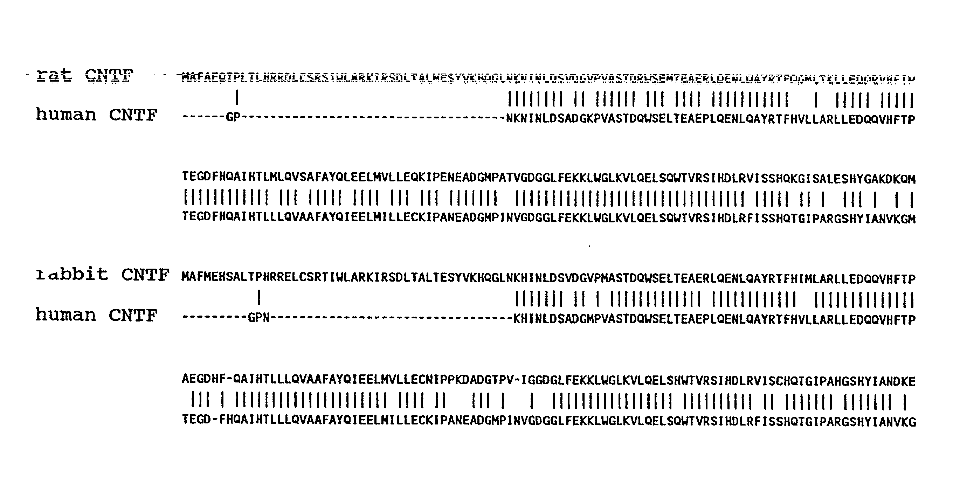

The amino acid sequence set forth in Figure 3 was arranged according to the method of Needleman-Wunsch with the rat and rabbit CNTF sequences (see Figure 4). The high homology of the obtained polypeptide sequence indicates with reasonable certainty that the isolated DNA segment corresponds to human CNTF.

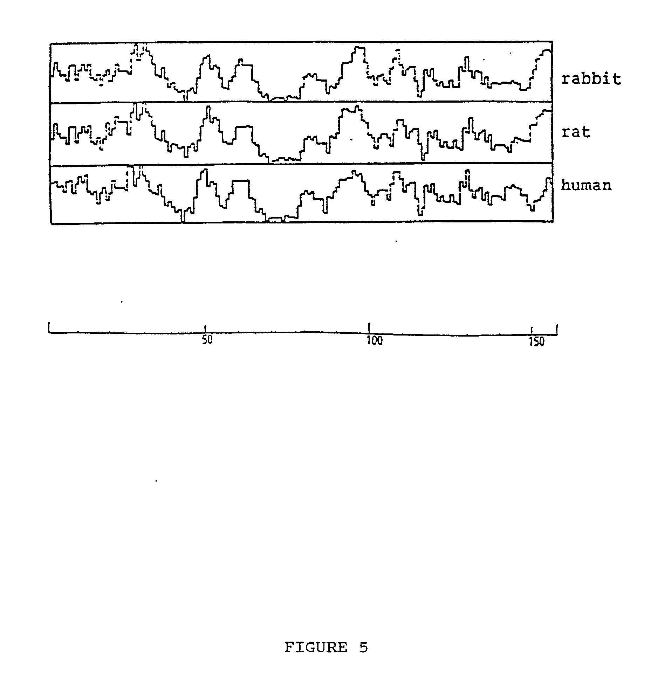

In addition, by comparing the hydropathicity profile of the segment cloned by the present inventors to that of the corresponding rabbit and rat segments (set forth in Figure 5), it can be demonstrated that, substantially, the amino acid residues changed in the polypeptide chain of the presently cloned segment do not affect the polypeptide's general profile. These two pieces of evidence substantially demonstrate that the presently cloned segment corresponds to the human ciliary neuronotrophic factor.

Example 2: Full Sequencing of the Human CNTF Gene

On the basis of the above described partial sequence for the human CNTF gene, it was possible to recover the complete gene segment. To do so, analyses were conducted on two commercially available, human gene libraries for the previously isolated CNTF portion.

The first is a human genome library of leukocytes in EMBL-3, and the other a human retina cDNA library in phage lambda gt11.

2. 1. Analysis of an EMBL-3 Library for human CNTF:

* with respect to conditions given in Gibbs, R.A., "DNA Amplification by the Polymerase Chain Reaction", Analytical Chemistry , 62, 202 (1990); Erlich, H., editor, "PCR Technology: Principles and Applications for DNA Amplification", Stockton Press, New York (1989); Innis, M.A., et al., editors, "PCR Protocols, A Guide to Methods and Applications", Academic Press, New York (1990).

Strategies for isolating a recombinant phage containing the DNA encoding the human ciliary neuronotrophic factor (CNTF) consist of determining a nucleotide homology with a radioactive probe prepared from the CNTF gene. Methodologies used to analyze the libraries are amply described in, for example, Sambrook J. et al (1989).

In particular, 800,000 recombinants are plated in four 20 x 20 cm plates and replications are effected with nitrocellulose. The nitrocellulose is prehybridized for 2 hours at 42° in 5 x SSC, 5 x Denhardt's and 50% Formamide. It is subsequently hybridized overnight in a solution analogous to that used for the prehybridization, plus 10% dextran sodium sulfate. The probe labelled with $^{32}$p is prepared with a Boehringer "random primer" kit from the Apal-Sall fragment of the pGEM7 plasmid described above. The nitrocellulose is washed four times at 50°C in 0.2 x SSC and 0.1% SDS for 30 minutes and then in 2 x SSC at room temperature. The four replications are then exposed to autoradiography using a Kodak XR-5 ray film and an intensifying screen. Twenty autoradiographic signals are obtained after hybridization which are taken to be CNTF clones. Each of these clones is examined for the presence of CNTF by the PCR technique and using oligonucleotides Apal and Sall described above. This analysis reveals the presence of 7 positive clones.

The clones were then examined for the presence of complete CNTF by the PCR technique. Two oligonucleotides are synthesized, the first based on the first nucleotides from rabbit CNTF, having the following sequence:

$$5'\text{AAC CATG GCTTTCATGGAGCTTCA3}'$$
$$\text{NcoI}$$

In this oligonucleotide some nucleotides at the 5' end are mutated to simplify cloning; namely, an Ncol site was added.

The second oligonucleotide is inserted in the human CNTF sequence directly upstream from a natural HindIII site to facilitate subsequent cloning, producing the following sequence:

$$5'\text{GATA AGCT TGAAGGTTCTCTTG3}'$$
$$\text{HindIII}$$

Amplification reveals the presence of a 1500bp band upon electrophoresis in an 1% Agarose gel. This band is purified, treated with T4 DNA kinase and then purified over Agarose and using GenecleanTM.

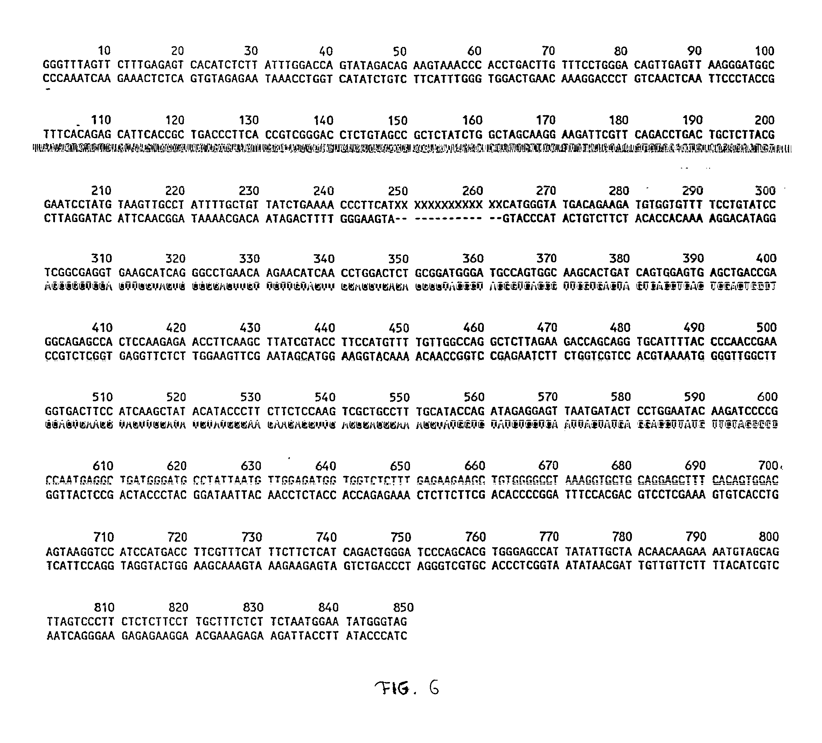

Subsequently, the purified DNA is digested with HindIII, is spliced and cloned in the pGEM7 plasmid between the Smal and HindIII sites, resulting in the plasmid pGEM7 CNTFh. This clone, sequenced by the method of Sanger (1983), reveals the presence of sequences analogous to those of human, rat and rabbit CNTF. The phage clone bearing this sequence is then purified to homogeneity, revealing an insert of 14Kb. The DNA sequences encoding CNTF contained in the recombinant phage are shown in Figure 6.

The gene sequence described in Figure 6 includes the gene portion described above and in Figure 1.

## 2.2. Analysis of a Library in Human Retina Phage For CNTF

Conventional methods of isolating a clone or clones encoding a specific protein from a library in gt 10 (Sambrook J. et al 1989) require the use of radioactive DNA probes which are homologous to the sequence to be isolated. If there is no homologous DNA probe, antibodies for the protein in question can be used. The library to be constructed and from which to isolate the clone is an expression library, e.g. gt11. With the advent of new techniques, the strategy of screening a library using radioactive DNA probes can be carried out by using PCR.

In the present case, having isolated the genomic sequence of CNTF and the sequence thus being known to the present inventors, two oligonucleotides were constructed to recover the gene without adding nucleotides to the 5' and 3' end of the gene itself. A first oligonucleotide called Ncol is located at the 5' end of the gene and has the following sequence:

AGC CATG GCTTTCACAGAGCAT

NcoI

which contains a natural NcoI restriction site facilitating its subsequent cloning. The second oligonucleotide, called EcoRI, has the following sequence:

5' AGG AATT CTACATTTTCTTGTTGTT 3'

EcoRI

This oligonucleotide contains the restriction site EcoRI which does not naturally occur in the gene.

With these two oligonucleotides, a PCR amplification is conducted using 1 $\mu$l from a human retina library equivalent to $3 \times 10^6$ different clones (determined from the serial dilution of the library; the cycle settings are 30 and the technique used is as previously described). The amplified product is first treated with T4 DNA kinase and then purified over Agarose and using the GeneeleanTM Kit.

Subsequently, the purified DNA is digested with EcoRI, repurified over Agarose and using the GeneeleanTM Kit and cloned in the plasmid pGEM7 between the sites SmaI and EcoRI, resulting in the plasmid pGEM7CNTFh.

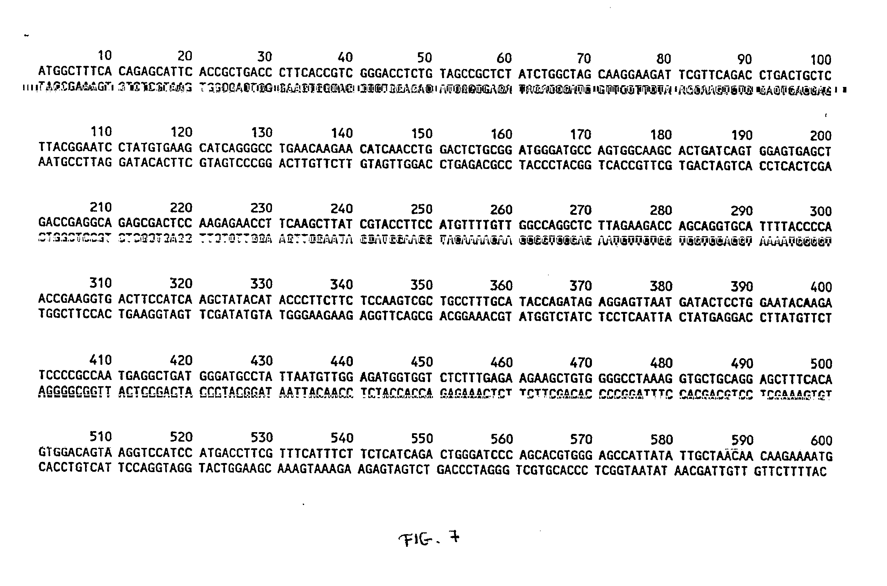

The sequence of this clone is shown in Figure 7. With regard to the genomic clone it shows a splice site 114bp after the start codon.

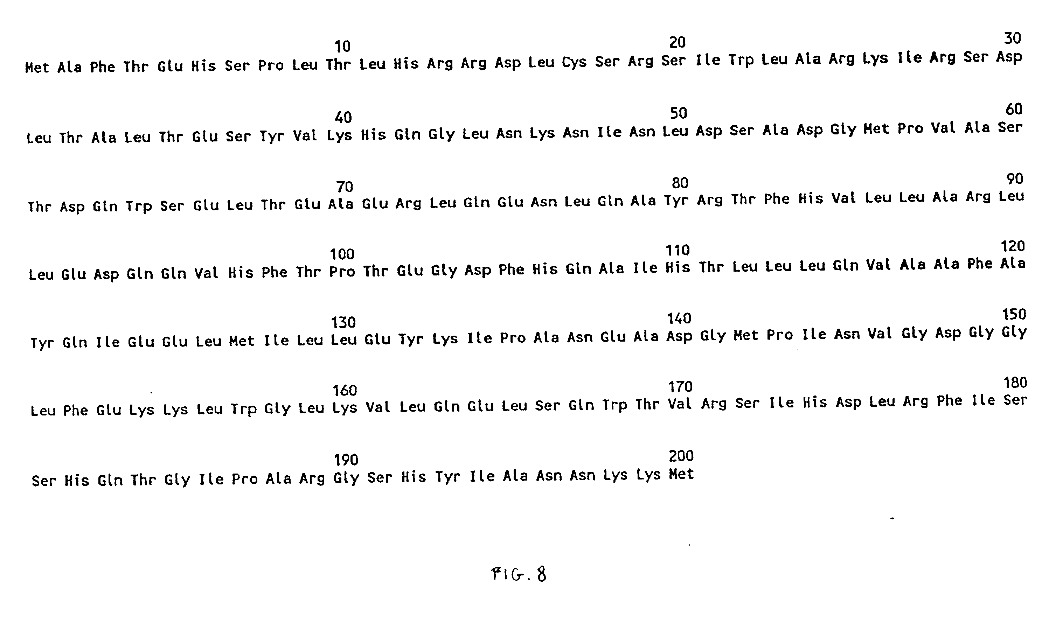

A comparison of the sequences shown in Figures 6 and 7 reveals an identical correspondence between the sequence reported in Figure 7 with the sequences 96-210 and 311-794 of Figure 6. On the basis of the information reported in Figures 6 and 7 with regard to the nucleotide sequences, the continuous amino acid sequence of the human CNTF protein is reported in Figure 8, wherein Met represents a methionine residue, Gln a glutamine residue, Asp an aspartic acid residue, Pro a proline residue, Tyr a tyrosine residue, Val a valine residue, Lys a lysine residue, Glu a glutamic acid residue, Ala an alanine residue, Asn an asparagine residue, Leu a leucine residue, Phe a phenylalanine residue, Gly a glycine residue, His a histidine residue, Ser a serine residue, Thr a threonine residue, Ile an isoleucine residue, Trp a tryptophan residue, Arg an arginine residue, and Cys a cysteine residue. The protein has a molecular weight of 22.8 kilodaltons and its iso-electric point is calculated to be 6.02.

Example 3: Prokaryotic Expression Vector for Human CNTF

The cDNA of the human CNTF cloned in the plasmid pGEM7 CNTFh between the SmaI and EcoRI sites is modified by the PCR technique to transcribe the protein more efficiently. A new oligonucleotide NcoI (2) is synthesized in which some nucleotides are changed so that the codons encoding the amino acids are those preferred by E.coli and other nucleotides are changed bearing in mind the stability of the messenger RNA in relation to the Shine-Dalgarno sequence the expression vector.

The sequence of this oligonucleotide is as follows:

AGC CATG GCTTTTACTGAACATTCA

NcoI

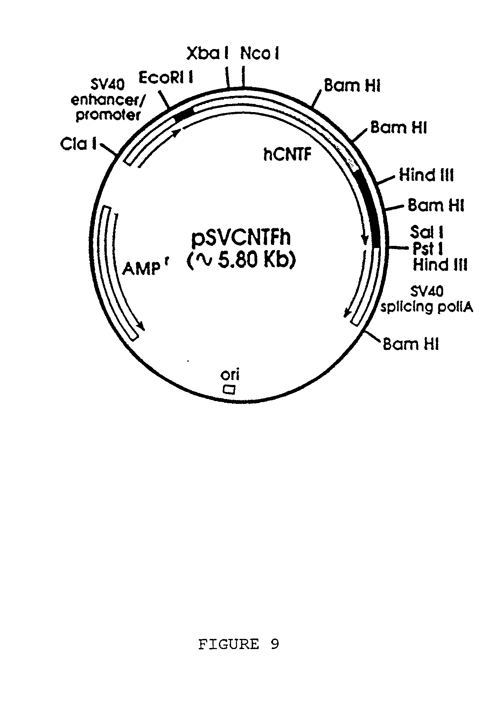

The amplified product obtained by PCR with the aforesaid oligonucleotides Nco1 (2) and EcoRI is cloned as previously described in the plasmid pJLA602 between the NcoI and EcoRI sites (Figure 9). The human CNTF is thus controlled by the lambda phage promoters $P_L$ and $P_R$ and by the $CI^{857}$ repressor.

This expression plasmid is transfected into various E. coli lines, including 71/18 and CAG628, to obtain expression of the protein.

Example 4: Bacterial Growth and Propagation

An E. coli colony of 71/18 containing the plasmid pJLA602CNTF is inoculated in 5 ml of LB medium containing 30 $\mu$g/ml of ampicillin. The cells are grown overnight at 30° C. An aliquot is then diluted at a ratio of 1/100 into M9 medium containing 20μg/ml of ampicillin. The cells are cultured in a fermenter to an absorbency of 0.4 OD at 550nm. The fermenter is then rapidly heated to 42°C. The cells are left to grow for another 3 hours after which they are centrifuged and resuspended in Tris/HCl pH8.0,10mM EDTA and are then ready for the subsequent extractions.

Example 5: Eukaryotic Expression Vector for Human CNTF

The sequence encoding human CNTF is inserted into a PSVT7 eukaryotic expression plasmid (Sambrook J. et al 1989). For this, DNA from the genomic EMBL-3 clone is used. Two new oligonucleotides are synthesized, the first at the 5' end and the second at the 3' end of the gene. The sequence of these oligonucleotides is as follows:

$$5' \; TTG \; AATT \; CATGGCTTTCACAGAGCAT \; 3'$$

$$EcoRI$$

$$5' \; TGG \; TCGA \; CTACATTTTCTTGTTGTT \; 3'$$

$$SalI$$

These two oligonucleotides also contain two restriction sites EcoRI and SalI which do not naturally occur in the gene to facilitate the subsequent cloning steps.

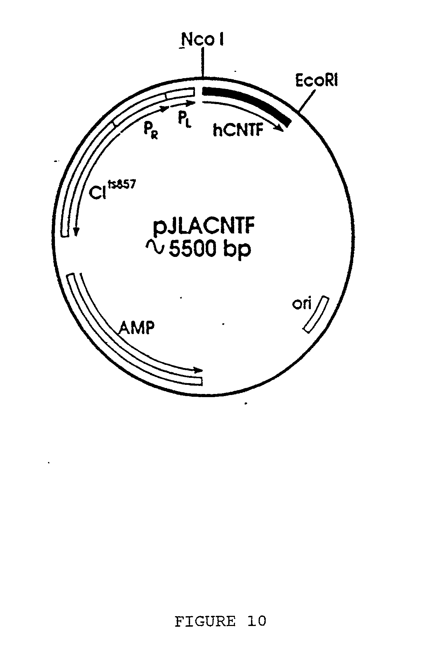

The amplified product obtained by PCR therefore contains the sequence encoding the first exon, the first intron and the sequence encoding the second exon. The fragment of about 1900bp is purified in Agarose and then in Geneclean and then cut with EcoRI and SalI and ligated into the pSVT7 vector between the EcoRI and SalI sites. The expression vector obtained is shown in Figure 10.

Example 6: Transient Transfection With Liposomes of Chinese Hamster Ovary (CHO) Cells

CHO cells are grown in a culture medium supplemented with 5% of fetal calf serum. The day before transfection they are treated with trypsin and replated so that by the next day they reach 80% confluence. The plasmid pSVhCNTF DNA is diluted to a concentration of 10 μg of DNA in 50 μl of H$_2$O to which 50 μl of LipofectinTM (GIBCO)are added, all contained in a polystyrene tube. After standing for 15 minutes this mixture is added to the cells which have previously been washed with OPTI-MEM medium (GIBCO). The cells are then incubated for 8 hours, and then the normal medium containing fetal calf serum is added to allow continuation of growth. The biological activity is assessed as described by Stöckli et al. (1989).

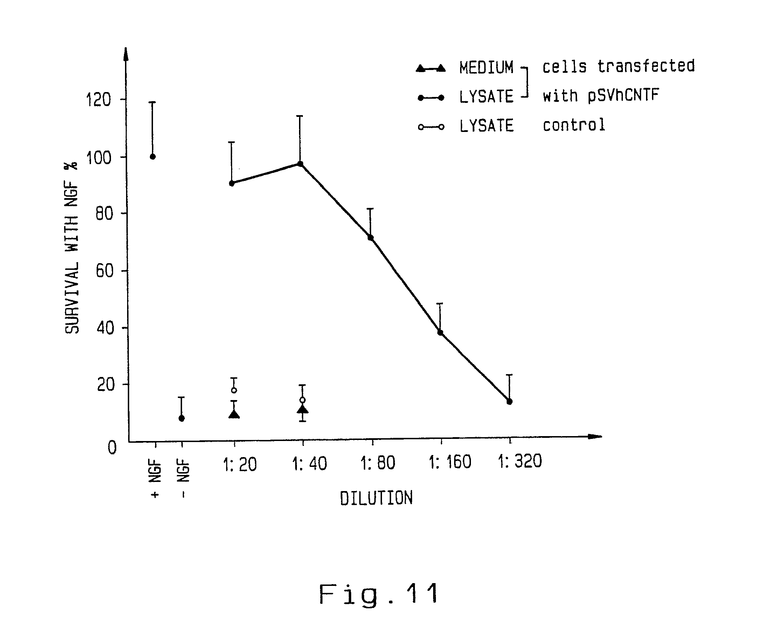

Ciliary chick ganglia are incubated with culture medium or with lysed CHO cells transfected with expression plasmids which do or do not bear human CNTF. Only the lysated CHO cells transfected with the plasmid pSVhCNTF, which bears the human CNTF gene, is able to maintain survival of the ciliary ganglia (Figure 11).

Example 7: Assessment of the Uniqueness of the Human CNTF Gene in the Human Genome

In order to establish whether the CNTF gene isolated and described herein is the only one in the human genome, a Southern blot was carried out with 10 μg samples per lane of DNA from human placenta digested with various restriction enzymes. The probe used is the same as that used for the detection of the human CNTF-DNA cloned first and described above which was labelled with $^{32}$P using a random primer kit from Boehringer. The digested DNA from human placenta is hybridized with the labelled probe having an overall activity 20 x 10[6] cpm and, once washed, exposed to autoradiography. After two days' exposure, single bands are visible when the DNA had been cut with HindIII, NcoI, EcoRI, XhoII, PstI, suggesting the presence of a single gene.

As discussed above, the present invention also relates to the therapeutic use of CNTF and its administration via pharmaceutical compositions containing the factor, either alone or together with other active substances. The CNTF is useful for the maintenance of or to prevent the loss of nervous function, and to treat the loss of nervous function due to acute or chronic pathological conditions, including the treatment of acute conditions such as cerebrovascular, infective, inflammatory, compressive and metabolic deficiencies, and chronic or neurodegenerative conditions. The CNTF is also useful for the treatment of neuropathological conditions caused by aging of the nervous system or diseases affecting the immune system.

The pharmaceutical preparations may contain, as the active substances, one or more combinations of the growth factor CNTF and a natural ganglioside (or gangliosides derivatives or semisynthetic analogues) or a salt or associations between these and other growth factors or polysaccharides (either natural, chemically modified or transformed into finished products such as biomaterials. Such pharmaceutical preparations can be for oral, topical, rectal, parenteral, local, inhalant or intracerebral use. They are therefore in solid or semisolid form, for example pills, tablets, creams, gelatin capsules, capsules, suppositories, soft gelatin capsules, gels, membranes, tubelets. For parenteral and intracerebral uses, those forms for intramuscular or subcutaneous administration can be used, or forms for infusion or intravenous or intracerebral injection and can therefore be prepared as solutions of the active compounds or as freeze-dried powders of the active compounds to be mixed with one or more pharmaceutically acceptable excipients or diluents, suitable for the aforesaid uses and with an osmolarity which is compatible with the physiological fluids. For local use, those preparations in the form of creams or ointments for topical use or in the form of sprays should be considered; for inhalant uses, preparations in the form of sprays, for example nose sprays, should be considered.

The preparations of the invention can be intended for administration to humans or animals. They contain preferably between 0.01% and 10% of active component in the case of solutions, sprays, ointments and creams and between 1% and 100% and preferably between 5% and 50% of active compound in the case of solid form preparations. Dosages to be administered depend on individual needs, on the desired effect and on the chosen route of administration, but daily dosages to humans by subcutaneous, intramuscular or intracerebral injection generally vary between 0.05 mg and 5 mg of active substance per kg of body weight.

The invention also embraces the therapeutic use of all the new complexes of gangliosides or hyaluronic acid derivatives or such derivatives with the growth factor CNTF for the aforesaid indications.

The pharmaceutical compositions containing the human CNTF molecule derived from recombinant DNA, without and possibly also with gangliosides, phospholipids, hyaluronic acid, can be prepared by per se known methods for the preparation of pharmaceutically acceptable compositions which can be administered to patients, and such that an effective quantity of the CNTF molecule is combined in a mixture with a pharmaceutically acceptable vehicle. Suitable vehicles and their formulation containing other proteins are described, for example, in "Remington's Pharmaceutical Sciences" (Remingtons's Pharmaceutical Sciences, Mack Publishing Company, Easton, Pa., USA 1985). These vehicles include injectable "deposit formulations".

On this basis, the pharmaceutical formulations include, albeit not exclusively, solutions of the CNTF growth factor or its freeze-dried powder in association with one or more pharmaceutically acceptable vehicles or diluents, and contained in buffered solutions with a suitable pH and iso-osmotic with the physiological fluids. In the case of freeze-dried preparations, supporting excipients are, for example, mannitol or glycin, and suitable buffered solutions of the desired volume can be supplied to obtain suitable isotonic buffered solutions with the desired pH. Similar solutions can be used for the pharmaceutical compositions of the CNTF molecule obtained from recombinant DNA in isotonic solutions of the desired volume, and include, but not exclusively, buffered saline solutions with phosphate or citrate at suitable concentrations so as to obtain isotonic pharmaceutical preparations of the desired pH, for example, neutral pH.

The pharmaceutical formulations may also include suppositories for rectal administration with lipophilic excipients, for example, hydrosoluble, self-emulsifying excipients such as glycogelatin or others. In these preparations, the CNTF growth factor obtained from recombinant DNA can be present in quantities varying between 0.01% and 1% in weight of the total excipient. The suppositories can contain, but are not limited to these, suitable quantities of acetylsalicylate.

For purely descriptive and not limiting purposes, we report hereafter some examples of pharmaceutical compositions prepared according to the present invention.

## A)   EXAMPLES OF INJECTABLE SOLUTIONS

Preparation No. 1 - one 2-ml vial contains:

| | | |
|---|---|---|
| Active substance | µg | 1 (3,200 BU) |
| Sodium chloride | mg | 16 |
| Citrate buffer pH = 7 | ml | 2 |
| in water for injection | | |

Preparation No. 2 - one 2-ml vial contains:

| | | |
|---|---|---|
| Active substance | µg | 10 (32,000 BU) |
| Sodium chloride | mg | 16 |
| Citrate buffer pH =7 | ml | 2 |
| in water for injection | | |

Preparation No. 3 - one 2-ml vial contains:

| | | |
|---|---|---|
| Active substance | µg | 1 (3,200 BU) |
| Sodium chloride | mg | 16 |
| Gangliosides as sodium salts | mg | 100 |
| Citrate buffer pH = 7 | ml | 2 |
| in water for injection | | |

Preparation No. 4 - one 2-ml vial contains:

| | | |
|---|---|---|
| Active substance | µg | 10 (32,000 BU) |
| Sodium chloride | mg | 16 |
| Gangliosides as sodium salts | mg | 50 |
| Citrate buffer pH = 7 | ml | 2 |
| in water for injection | | |

Preparation No. 5 - one 2-ml vial contains:

| | | |
|---|---|---|
| Active substance | µg | 1 (3,200 BU) |
| Sodium chloride | mg | 16 |
| Monosialotetrahexosylganglioside (GM$_1$) sodium salts | mg | 100 |

12

```
Citrate buffer pH = 7                          ml   2
in water for injection


Preparation No. 6 - one 2-ml vial contains:
Active substance                          µg   10 (32,000 BU)
Sodium chloride                           mg   16
Monosialotetrahexosylganglioside
(GM₁) sodium salts                        mg  100
Citrate buffer pH = 7                     ml   2
in water for injection


Preparation No. 7
a)   one 2-ml ampoule contains:
Freeze-dried active substance            µg   4 (12,800 BU)
Glycine                                  mg   30
b)   one 2-ml ampoule of solvent contains:
Sodium chloride                          mg   16
Citrate buffer in water                  ml   2
Water for injection


Preparation No. 8
a)   one 2-ml vial contains:
Freeze-dried active substance            µg   4 (12,800 BU)
Mannitol                                 mg   40
b)   one 2-ml ampoule of solvent contains:
Sodium chloride                          mg   16
Citrate buffer in water                  ml   2
Water for injection
```

Preparation No. 9

a)   one 3-ml vial contains:

| Freeze-dried active substance | µg | 10 (32,000 BU) |
| Glycine | mg | 45 |

b)   one 3-ml ampoule of solvent contains:

| Sodium chloride | mg | 24 |
| Citrate buffer in water | ml | 3 |

Water for injection

Preparation No. 10

a)   one 3-ml vial contains:

| Freeze-dried active substance | µg | 10 (32,000 BU) |
| Gangliosides as sodium salts | mg | 100 |
| Glycine | mg | 45 |

b)   one 3-ml ampoule of solvent contains:

| Sodium chloride | mg | 24 |
| Citrate buffer in water | ml | 3 |

Water for injection

Preparation No. 11

a)   one 3-ml vial contains:

| Freeze-dried active substance | µg | 10 (32,000 BU) |
| Gangliosides as sodium salts | mg | 50 |
| Glycine | mg | 45 |

b)   one 3-ml ampoule of solvent contains:

| Sodium chloride | mg | 24 |
| Citrate buffer in water | ml | 3 |

Water for injection

Preparation No. 12

a)   one 3-ml vial contains:

| Freeze-dried active substance | µg | 1 (3,200 BU) |

Monosialotetrahexosylganglioside

(GM$_1$) sodium salts                             mg  100

Glycine                                           mg   45

b)   <u>one 3-ml ampoule of solvent contains</u>:

Sodium chloride                                   mg   24

Citrate buffer in water                           ml    3

Water in injection


<u>Preparation No. 13</u>

a)   <u>one 3-ml vial contains</u>:

Freeze-dried active substance        µg   10 (32,000 BU)

Monosialotetrahexosylganglioside

(GM$_1$) sodium salts                             mg  100

Glycine                                           mg   45

b)   <u>One 3-ml ampoule of solvent contains</u>:

Sodium chloride                                   mg   24

Citrate buffer in water                           ml    3

Water for injection


<u>Preparation No. 14</u>

a)   <u>one 3-ml vial contains</u>:

Freeze-dried active substance        µg   10 (32,000 BU)

3-sn-phosphatidyl)L-serine                        mg   50

Lecithin                                          mg   15

Mannitol                                          mg  100

b)   <u>One 4-ml ampoule of solvent contains</u>:

Mannitol                                          mg   60

Water for injection to vol.                       ml    4


<u>Preparation No. 15</u>

a)   <u>one 3-ml ampoule contains</u>:

Freeze-dried active substance        µg   10 (32,000 BU)

Mannitol                                          mg   60

b)   one 3-ml ampoule of solvent contains:

| | | |
|---|---|---|
| Sodium chloride | mg | 24 |
| Citrate buffer in water | ml | 3 |
| Water for injection | | |

B)   EXAMPLES FOR SUBCUTANEOUS INJECTION

Preparation No. 16

a)   one 2-ml vial contains:

| | | |
|---|---|---|
| Freeze-dried active substance | µg | 5 (16,000 BU) |
| Mannitol | mg | 30 |

b)   one 2-ml ampoule of solvent contains:

| | | |
|---|---|---|
| Sodium chloride | mg | 16 |
| Citrate buffer in water | ml | 2 |
| Water for injection | | |

C)   EXAMPLES OF SUPPOSITORIES FOR THE RECTAL ROUTE

Preparation No. 17

| | | |
|---|---|---|
| Active substance | µg | 10 (32,000 BU) |
| Cocoa butter | mg | 2.5 |

Preparation No. 18

| | | |
|---|---|---|
| Active substance | µg | 10 (32,000 BU) |
| Carbowax 1540 | g | 1.75 |
| Carbowax 6000 | g | 0.75 |

Preparation No. 19

| | | |
|---|---|---|
| Active substance | µg | 10 (32,000 BU) |
| Tween 61 | g | 2.125 |
| Lanolin | g | 0.25 |

### Preparation No. 20

| | | |
|---|---|---|
| Active substance | µg | 10 (32,000 BU) |
| Glycerine | g | 1.5 |
| Water | g | 0.25 |
| Gelatine | g | 0.25 |

The invention being thus described, it is clear that these methods can be modified in various ways. Such modifications are not to be considered as divergences from the spirit and purposes of the invention, and any modification which appears evident to an expert in the art comes within the scope of the following claims:

**Claims**

1. A DNA isolate which comprises a DNA sequence encoding human ciliary neuronotrophic factor.

2. The DNA isolate according to claim 1, wherein said DNA comprises the following nucleotide sequence;

```
GGGTTTAGTT  CTTTGAGAGT  CACATCTCTT  ATTTGGACCA  GTATAGACAG
AAGTAAACCC  ACCTGACTTG  TTTCCTGGGA  CAGTTGAGTT  AAGGGATGGC
TTTCACAGAG  CATTCACCGC  TGACCCTTCA  CCGTCGGGAC  CTCTGTAGCC
GCTCTATCTG  GCTAGCAAGG  AAGATTCGTT  CAGACCTGAC  TGCTCTTACG
GAATCCTATG  TAAGTTGCCT  ATTTTGCTGT  TATCTGAAAA  CCCTTCATXX
XXXXXXXXXX  XXCATGGGTA  TGACAGAAGA  TGTGGTGTTT  TCCTGTATCC
TCGGCGAGGT  GAAGCATCAG  GGCCTGAACA  AGAACATCAA  CCTGGACTCT
GCGGATGGGA  TGCCAGTGGC  AAGCACTGAT  CAGTGGAGTG  AGCTGACCGA
GGCAGAGCCA  CTCCAAGAGA  ACCTTCAAGC  TTATCGTACC  TTCCATGTTT
TGTTGGCCAG  GCTCTTAGAA  GACCAGCAGG  TGCATTTTAC  CCCAACCGAA
GGTGACTTCC  ATCAAGCTAT  ACATACCCTT  CTTCTCCAAG  TCGCTGCCTT
TGCATACCAG  ATAGAGGAGT  TAATGATACT  CCTGGAATAC  AAGATCCCCG
CCAATGAGGC  TGATGGGATG  CCTATTAATG  TTGGAGATGG  TGGTCTCTTT
GAGAAGAAGC  TGTGGGGCCT  AAAGGTGCTG  CAGGAGCTTT  CACAGTGGAC
AGTAAGGTCC  ATCCATGACC  TTCGTTTCAT  TTCTTCTCAT  CAGACTGGGA
TCCCAGCACG  TGGGAGCCAT  TATATTGCTA  ACAACAAGAA  AATGTAGCAG
TTAGTCCCTT  CTCTCTTCCT  TGCTTTCTCT  TCTAATGGAA  TATGGGTAG
```

or variants thereof in accordance with the degeneracy of the Genetic Code.

3. The DNA isolate according to claim 1, wherein said DNA comprises the following nucleotide sequence:

```
ATGGCTTTCA CAGAGCATTC ACCGCTGACC CTTCACCGTC GGGACCTCTG
TAGCCGCTCT ATCTGGCTAG CAAGGAAGAT TCGTTCAGAC CTGACTGCTC
TTACGGAATC CTATGTGAAG CATCAGGGCC TGAACAAGAA CATCAACCTG
GACTCTGCGG ATGGGATGCC AGTGGCAAGC ACTGATCAGT GGAGTGAGCT
GACCGAGGCA GAGCGACTCC AAGAGAACCT TCAAGCTTAT CGTACCTTCC
ATGTTTTGTT GGCCAGGCTC TTAGAAGACC AGCAGGTGCA TTTTACCCCA
ACCGAAGGTG ACTTCCATCA AGCTATACAT ACCCTTCTTC TCCAAGTCGC
TGCCTTTGCA TACCAGATAG AGGAGTTAAT GATACTCCTG GAATACAAGA
TCCCCGCCAA TGAGGCTGAT GGGATGCCTA TTAATGTTGG AGATGGTGGT
CTCTTTGAGA AGAAGCTGTG GGGCCTAAAG GTGCTGCAGG AGCTTTCACA
GTGGACAGTA AGGTCCATCC ATGACCTTCG TTTCATTTCT TCTCATCAGA
CTGGGATCCC AGCACGTGGG AGCCATTATA TTGCTAACAA CAAGAAAATG
```

or variants thereof in accordance with the degeneracy of the Genetic Code.

4. The DNA isolate according to claim 1, wherein said DNA isolate encodes the following amino acid sequence:

```
Met Ala Phe Thr Glu His Ser Pro Leu Thr Leu His Arg
Arg Asp Leu Cys Ser Arg Ser Ile Trp Leu Ala Arg Lys
Ile Arg Ser Asp Leu Thr Ala Leu Thr Glu Ser Tyr Val
Lys His Gln Gly Leu Asn Lys Asn Ile Asn Leu Asp Ser
Ala Asp Gly Met Pro Val Ala Ser Thr Asp Gln Trp Ser
Glu Leu Thr Glu Ala Glu Arg Leu Gln Glu Asn Leu Gln
Ala Tyr Arg Thr Phe His Val Leu Leu Ala Arg Leu Leu
```

```
Glu Asp Gln Gln Val His Phe Thr Pro Thr Glu Gly Asp
Phe His Gln Ala Ile His Thr Leu Leu Leu Gln Val Ala
Ala Phe Ala Tyr Gln Ile Glu Glu Leu Met Ile Leu Leu
Glu Tyr Lys Ile Pro Ala Asn Glu Ala Asp Gly Met Pro
Ile Asn Val Gly Asp Gly Gly Leu Phe Glu Lys Lys Leu
Trp Gly Leu Lys Val Leu Gln Glu Leu Ser Gln Trp Thr
Val Arg Ser Ile His Asp Leu Arg Phe Ile Ser Ser His
Gln Thr Gly Ile Pro Ala Arg Gly Ser His Tyr Ile Ala
Asn Asn Lys Lys Met
```

5. A recombinant expression vector containing a DNA sequence according to any one of claims 1 to 4, wherein said vector is capable of expressing human ciliary neuronotrophic factor in a transformed microorganism or in cell culture.

6. A microorganism transformed with a vector according to claim 5 and being capable of expressing human ciliary neuronotrophic factor.

7. The microorganism according to claim 6 which is E. coli.

8. A cell culture transformed with a vector according to claim 5 and being capable of expressing human ciliary neuronotrophic factor.

9. The cell culture according to claim 8, wherein the cell line is a non-human mammalian cell line.

10. The cell culture according to claim 9, wherein said cell line is a Chinese Hamster Ovary cell line.

11. Substantially pure human ciliary neuronotrophic factor which is free from contaminant proteins of human origin.

12. The substantially pure human ciliary neuronotrophic factor according to claim 11, which has the following amino acid sequence:

```
Met Ala Phe Thr Glu His Ser Pro Leu Thr Leu His Arg
Arg Asp Leu Cys Ser Arg Ser Ile Trp Leu Ala Arg Lys
Ile Arg Ser Asp Leu Thr Ala Leu Thr Glu Ser Tyr Val
Lys His Gln Gly Leu Asn Lys Asn Ile Asn Leu Asp Ser
Ala Asp Gly Met Pro Val Ala Ser Thr Asp Gln Trp Ser
Glu Leu Thr Glu Ala Glu Arg Leu Gln Glu Asn Leu Gln
Ala Tyr Arg Thr Phe His Val Leu Leu Ala Arg Leu Leu
Glu Asp Gln Gln Val His Phe Thr Pro Thr Glu Gly Asp
Phe His Gln Ala Ile His Thr Leu Leu Leu Gln Val Ala
Ala Phe Ala Tyr Gln Ile Glu Glu Leu Met Ile Leu Leu
Glu Tyr Lys Ile Pro Ala Asn Glu Ala Asp Gly Met Pro
Ile Asn Val Gly Asp Gly Gly Leu Phe Glu Lys Lys Leu
Trp Gly Leu Lys Val Leu Gln Glu Leu Ser Gln Trp Thr
Val Arg Ser Ile His Asp Leu Arg Phe Ile Ser Ser His
Gln Thr Gly Ile Pro Ala Arg Gly Ser His Tyr Ile Ala
Asn Asn Lys Lys Met
```

13. A pharmaceutical composition containing the human ciliary neuronotrophic growth factor according to claim 11 or 12, optionally in combination with a pharmaceutically acceptable carrier or diluent.

14. The pharmaceutical composition according to claim 13 for the treatment of nervous disorders.

15. The pharmaceutical composition according to claim 13 for maintaining, preventing loss or recovering nervous function.

16. The pharmaceutical composition according to any one of claims 13 to 15, which further comprises a natural ganglioside, or a derivative, or a semisynthetic analogue, or a salt of such ganglioside.

17. The pharmaceutical composition according to any one of claims 13 to 16, which further comprises a natural polysaccharide, or a derivative, or a semisynthetic analogue of such polysaccharide.

18. The pharmaceutical composition according to claim 17, wherein said polysaccharide is hyaluronic acid.

19. Use of a compound according to claim 11 or 12 for the preparation of a pharmaceutical composition according to any one of claims 13 to 18.

20. Use according to claim 19, wherein the composition is useful for the treatment of nervous disorders by maintaining, preventing loss or recovering nervous function.

21. Use according to claim 19, wherein the pharmaceutical composition is useful for the treatment of neuropathological conditions caused by aging of the nervous system or diseases affecting the immune system.

EP 0 446 931 A1

```
         10         20         30         40         50         60         70         80         90        100
GGGCCCGAAC AAGAACATCA ACCTGGACTC TGCGGATGGG ATGCCAGTGG CAAGCACTGA TCAGTGGAGT GAGCTGACCG AGGCAGAGCC ACTCCAAGAG
CCCGGGCTTG TTCTTGTAGT TGGACCTGAG ACGCCTACCC TACGGTCACC GTTCGTGACT AGTCACCTCA CTCGACTGGC TCCGTCTCGG TGAGGTTCTC

        110        120        130        140        150        160        170        180        190        200
AACCTTCAAG CTTATCGTAC CTTCCATGTT TTGTTGGCCA GGCTCTTAGA AGACCAGCAG GTGCATTTTA CCCCAACCGA AGGTGACTTC CATCAAGCTA
TTGGAAGTTC GAATAGCATG GAAGGTACAA AACAACCGGT CCGAGAATCT TCTGGTCGTC CACGTAAAAT GGGGTTGGCT TCCACTGAAG GTAGTTCGAT

        210        220        230        240        250        260        270        280        290        300
TACATACCCT TCTTCTCCAA GTCGCTGCCT TTGCATACCA GATAGAGGAG TTAATGATAC TCTTGGAATG CAAGATCCCT GCCAATGAAG CTGATGGGAT
ATGTATGGGA AGAAGAGGTT CAGCGACGGA AACGTATGGT CTATCTCCTC AATTACTATG AGAACCTTAC GTTCTAGGGA CGGTTACTTC GACTACCCTA

        310        320        330        340        350        360        370        380        390        400
GCCTATTAAT GTTGGAGATG GTGGTCTCTT TGAGAAGAAG CTGTGGGGCC TAAAGGTGCT GCAGGAGCTT TCACAGTGGA CAGTAAGGTC CATCCATGAC
CGGATAATTA CAACCTCTAC CACCAGAGAA ACTCTTCTTC GACACCCCGG ATTTCCACGA CGTCCTCGAA AGTGTCACCT GTCATTCCAG GTAGGTACTG

        410        420        430        440        450        460        470        480        490        500
CTTCGTTTCA TTTCTTCTCA TCAGACTGGG ATCCCAGCAC GTGGGAGCCA TTATATTGCT AACGTCAAAG GAATGTAGTC GA
GAAGCAAAGT AAAGAAGAGT AGTCTGACCC TAGGGTCGTG CACCCTCGGT AATATAACGA TTGCAGTTTC CTTACATCAG CT
```

FIG. 1

```
G P E Q E H Q P G L C G W D A S G K H * S V E * A D R G R A T P R E P S S L S Y
G P N K N I N L D S A D S M P V A S T D Q W S E L T E A E P L Q E N L Q A Y R T
  A R T R T S T W T L R M G C Q W Q A L I S G V S * P R Q S H S K R T F K L I V P
GGGCCCGAACAAGAACATCAACCTGGACTCTGCGGATGGGATGCCAGTGGCAAGCACTGATCAGTGGAGTGAGCTGACCGAGGCAGAGCCACTCCAAGAGAACCTTCAAGCTTATCGTAC


L P C F V G Q A L R R P A G A F Y P N R R * L P S S Y T Y P S S P S R C L C I P
F H V L L A R L L E D Q Q V H F T P T E G D F H Q A I H T L L L Q V A A F A Y Q
  S M F C W P G S * K T S R C I L P Q P K V T S I K L Y I P F F S K S L P L H T R
CTTCCATGTTTTGTTGGCCAGGCTCTTAGAAGACCAGCAGGTGCATTTTACCCCAACCGAAGGTGACTTCCATCAAGCTATACATACCCTTCTTCTCCAAGTCGCTGCCTTTGCATACCA


D R G V N D T L G M Q D P C Q * S * W D A Y * C W R W W S L * E E A V G P K G A
I E E L M I L L E C K I P A N E A D G M P I N V G D G G L F E K K L W G L K V L
  * R S * * Y S W N A R S L P M K L M G C L L M L E M V V S L R R S C G A * R C C
GATAGAGGAGTTAATGATACTCTTGGAATGCAAGATCCCTGCCAATGAAGCTGATGGGATGCCTATTAATGTTGGAGATGGTGGTCTCTTTGAGAAGAAGCTGTGGGGCCTAAAGGTGCT


A G A F T V D S K V H P * P S F H F F S S D W D P S T W E P L Y C * R Q R N V V
Q E L S Q W T V R S I H D L R F I S S H Q T G I P A R G S H Y I A N V K G M * S
  R S F H S G Q * G P S M T F V S F L L I R L G S Q H V G A I I L L T S K E C S R
GCAGGAGCTTTCACAGTGGACAGTAAGGTCCATCCATGACCTTCGTTTCATTTCTTCTCATCAGACTGGGATCCCAGCACGTGGGAGCCATTATATTGCTAACGTCAAAGGAATGTAGTC
```

FIG. 2

EP 0 446 931 A1

EP 0 446 931 A1

```
                    10                                      20                                      30
Gly Pro Asn Lvs Asn ile Asn Leu Asp Ser Ala Asp Gly Met Pro Val Ala Ser Thr Asp Gin Trp Ser Glu Leu Thr Glu Ala Glu Pro


                    40                                      50                                      60
Leu Gln Glu Asp Leu Gln Ala Tyr Arg Thr Phe His Val Leu Leu Ala Arg Leu Leu Glu Asp Gln Gln Val His Phe Thr Pro Thr Glu


                    70                                      80                                      90
Gly Asp Phe His Gln Ala Ile His Thr Leu Leu Leu Gln Val Ala Ala Phe Ala Tyr Gln Ile Glu Glu Leu Met Ile Leu Leu Glu Cys


                    100                                     110                                     120
Lys Ile Pro Ala Asn Glu Ala Asp Gly Met Pro Ile Asn Val Gly Asp Gly Gly Leu Phe Glu Lys Lys Leu Trp Gly Leu Lys Val Leu


                    130                                     140                                     150
Gln Glu Leu Ser Gln Trp Thr Val Arg Ser Ile His Asp Leu Arg Phe Ile Ser Ser His Gln Thr Gly Ile Pro Ala Arg Gly Ser His


                    160
Tyr Ile Ala Asn Val Lys Gly Met
```

FIG. 3

```
rat CNTF      MAFAEDTPLTLHRRDLCSRSIWLARKIRSDLTALMESYVKHQGLNKNINLDSVDGVPVASTDRWSEMTEAERLQENLQAYRTFQGMLTKLLEDQQVHFTP
                    |                               |||||||| || |||||| ||| |||| ||||||||||| | ||||| |||||
human CNTF    ------GP----------------------------------NKNINLDSADGKPVASTDQWSELTEAEPLQENLQAYRTFHVLLARLLEDQQVHFTP


              TEGDFHQAIHTLMLQVSAFAYQLEELMVLLEQKIPENEADGMPATVGDGGLFEKKLWGLKVLQELSQWTVRSIHDLRVISSHQKGISALESHYGAKDKQM
              |||||||||||| ||| |||||| |||| ||| ||| |||||||| |||||||||||||||||||||||||||||||||||||| ||||| || | ||| | | |
              TEGDFHQAIHTLLLQVAAFAYQIEELMILLECKIPANEADGMPINVGDGGLFEKKLWGLKVLQELSQWTVRSIHDLRFISSHQTGIPARGSHYIANVKGM


rabbit CNTF   MAFMEHSALTPHRRELCSRTIWLARKIRSDLTALTESYVKHQGLNKHINLDSVDGVPMASTDQWSELTEAERLQENLQAYRTFHIMLARLLEDQQVHFTP
                    |                           ||||||| || | ||||||||||||| ||||||||||| |||||||||||||
human CNTF    ---------GPN-------------------------------KHINLDSADGMPVASTDQWSELTEAEPLQENLQAYRTFHVLLARLLEDQQVHFTP


              AEGDHF-QAIHTLLLQVAAFAYQIEELMVLLECNIPPKDADGTPV-IGGDGLFEKKLWGLKVLQELSHWTVRSIHDLRVISCHQTGIPAHGSHYIANDKE
              ||| | ||||||||||||||||||||| |||| || ||| | | ||||||||||||||| ||||||||| || ||||||| |||||||| |
              TEGD-FHQAIHTLLLQVAAFAYQIEELMILLECKIPANEADGMPINVGDGGLFEKKLWGLKVLQELSQWTVRSIHDLRFISSHQTGIPARGSHYIANVKG
```

rabbit

rat

human

FIGURE 5

EP 0 446 931 A1

```
        10         20         30         40         50         60         70         80         90        100
GGGTTTAGTT CTTTGAGAGT CACATCTCTT ATTTGGACCA GTATAGACAG AAGTAAACCC ACCTGACTTG TTTCCTGGGA CAGTTGAGTT AAGGGATGGC
CCCAAATCAA GAAACTCTCA GTGTAGAGAA TAAACCTGGT CATATCTGTC TTCATTTGGG TGGACTGAAC AAAGGACCCT GTCAACTCAA TTCCCTACCG
-

       110        120        130        140        150        160        170        180        190        200
TTTCACAGAG CATTCACCGC TGACCCTTCA CCGTCGGGAC CTCTGTAGCC GCTCTATCTG GCTAGCAAGG AAGATTCGTT CAGACCTGAC TGCTCTTACG


       210        220        230        240        250        260        270        280        290        300
GAATCCTATG TAAGTTGCCT ATTTTGCTGT TATCTGAAAA CCCTTCATXX XXXXXXXXXX XXCATGGGTA TGACAGAAGA TGTGGTGTTT TCCTGTATCC
CTTAGGATAC ATTCAACGGA TAAAACGACA ATAGACTTTT GGGAAGTA-- ---------- --GTACCCAT ACTGTCTTCT ACACCACAAA AGGACATAGG


       310        320        330        340        350        360        370        380        390        400
TCGGCGAGGT GAAGCATCAG GGCCTGAACA AGAACATCAA CCTGGACTCT GCGGATGGGA TGCCAGTGGC AAGCACTGAT CAGTGGAGTG AGCTGACCGA


       410        420        430        440        450        460        470        480        490        500
GGCAGAGCCA CTCCAAGAGA ACCTTCAAGC TTATCGTACC TTCCATGTTT TGTTGGCCAG GCTCTTAGAA GACCAGCAGG TGCATTTTAC CCCAACCGAA
CCGTCTCGGT GAGGTTCTCT TGGAAGTTCG AATAGCATGG AAGGTACAAA ACAACCGGTC CGAGAATCTT CTGGTCGTCC ACGTAAAATG GGGTTGGCTT


       510        520        530        540        550        560        570        580        590        600
GGTGACTTCC ATCAAGCTAT ACATACCCTT CTTCTCCAAG TCGCTGCCTT TGCATACCAG ATAGAGGAGT TAATGATACT CCTGGAATAC AAGATCCCCG


       610        620        630        640        650        660        670        680        690        700
CCAATGAGGC TGATGGGATG CCTATTAATG TTGGAGATGG TGGTCTCTTT GAGAAGAAGC TGTGGGGCCT AAAGGTGCTG CAGGAGCTTT CACAGTGGAC
GGTTACTCCG ACTACCCTAC GGATAATTAC AACCTCTACC ACCAGAGAAA CTCTTCTTCG ACACCCCGGA TTTCCACGAC GTCCTCGAAA GTGTCACCTG


       710        720        730        740        750        760        770        780        790        800
AGTAAGGTCC ATCCATGACC TTCGTTTCAT TTCTTCTCAT CAGACTGGGA TCCCAGCACG TGGGAGCCAT TATATTGCTA ACAACAAGAA AATGTAGCAG
TCATTCCAGG TAGGTACTGG AAGCAAAGTA AAGAAGAGTA GTCTGACCCT AGGGTCGTGC ACCCTCGGTA ATATAACGAT TGTTGTTCTT TTACATCGTC


       810        820        830        840        850
TTAGTCCCTT CTCTCTTCCT TGCTTTCTCT TCTAATGGAA TATGGGTAG
AATCAGGGAA GAGAGAAGGA ACGAAAGAGA AGATTACCTT ATACCCATC
```

FIG. 6

EP 0 446 931 A1

```
        10         20         30         40         50         60         70         80         90        100
ATGGCTTTCA CAGAGCATTC ACCGCTGACC CTTCACCGTC GGGACCTCTG TAGCCGCTCT ATCTGGCTAG CAAGGAAGAT TCGTTCAGAC CTGACTGCTC
TACCGAAAGT GTGTCGTAAG TGGCACTGGG CAAGTGGCAG CCCTGGAGAC ATCGGCGAGA TAGACCGATC AGCAAGTCTG AGCAAGTCTG GACTGACGAG

       110        120        130        140        150        160        170        180        190        200
TTACGGAATC CTATGTGAAG CATCAGGGCC TGAACAAGAA CATCAACCTG GACTCTGCGG ATGGGATGCC AGTGGCAAGC ACTGATCAGT GGAGTGAGCT
AATGCCTTAG GATACACTTC GTAGTCCCGG ACTTGTTCTT GTAGTTGGAC CTGAGACGCC TACCCTACGG TCACCGTTCG TGACTAGTCA CCTCACTCGA

       210        220        230        240        250        260        270        280        290        300
GACCGAGGCA GAGCGACTCC AAGAGAACCT TCAAGCTTAT CGTACCTTCC ATGTTTTGTT GGCCAGGCTC TTAGAAGACC AGCAGGTGCA TTTTACCCCA
CTGGCTCCGT CTCGCTGAGG TTCTCTTGGA AGTTCGAATA GCATGGAAGG TACAAAACAA CCGGTCCGAG AATCTTCTGG TCGTCCACGT AAAATGGGGT

       310        320        330        340        350        360        370        380        390        400
ACCGAAGGTG ACTTCCATCA AGCTATACAT ACCCTTCTTC TCCAAGTCGC TGCCTTTGCA TACCAGATAG AGGAGTTAAT GATACTCCTG GAATACAAGA
TGGCTTCCAC TGAAGGTAGT TCGATATGTA TGGGAAGAAG AGGTTCAGCG ACGGAAACGT ATGGTCTATC TCCTCAATTA CTATGAGGAC CTTATGTTCT

       410        420        430        440        450        460        470        480        490        500
TCCCCGCCAA TGAGGCTGAT GGGATGCCTA TTAATGTTGG AGATGGTGGT CTCTTTGAGA AGAAGCTGTG GGGCCTAAAG GTGCTGCAGG AGCTTTCACA
AGGGGCGGTT ACTCCGACTA CCCTACGGAT AATTACAACC TCTACCACCA GAGAAACTCT TCTTCGACAC CCCGGATTTC CACGACGTCC TCGAAAGTGT

       510        520        530        540        550        560        570        580        590        600
GTGGACAGTA AGGTCCATCC ATGACCTTCG TTTCATTTCT TCTCATCAGA CTGGGATCCC AGCACGTGGG AGCCATTATA TTGCTAACAA CAAGAAAATG
CACCTGTCAT TCCAGGTAGG TACTGGAAGC AAAGTAAAGA AGAGTAGTCT GACCCTAGGG TCGTGCACCC TCGGTAATAT AACGATTGTT GTTCTTTTAC
```

FIG. 7

10                     20                    30

Met Ala Phe Thr Glu His Ser Pro Leu Thr Leu His Arg Arg Asp Leu Cys Ser Arg Ser Ile Trp Leu Ala Arg Lys Ile Arg Ser Asp

40                     50                    60

Leu Thr Ala Leu Thr Glu Ser Tyr Val Lys His Gln Gly Leu Asn Lys Asn Ile Asn Leu Asp Ser Ala Asp Gly Met Pro Val Ala Ser

70                     80                    90

Thr Asp Gln Trp Ser Glu Leu Thr Glu Ala Glu Arg Leu Gln Glu Asn Leu Gln Ala Tyr Arg Thr Phe His Val Leu Leu Ala Arg Leu

100                    110                  120

Leu Glu Asp Gln Gln Val His Phe Thr Pro Thr Glu Gly Asp Phe His Gln Ala Ile His Thr Leu Leu Leu Gln Val Ala Ala Phe Ala

130                    140                  150

Tyr Gln Ile Glu Glu Leu Met Ile Leu Leu Glu Tyr Lys Ile Pro Ala Asn Glu Ala Asp Gly Met Pro Ile Asn Val Gly Asp Gly Gly

160                    170                  180

Leu Phe Glu Lys Lys Leu Trp Gly Leu Lys Val Leu Gln Glu Leu Ser Gln Trp Thr Val Arg Ser Ile His Asp Leu Arg Phe Ile Ser

190                    200

Ser His Gln Thr Gly Ile Pro Ala Arg Gly Ser His Tyr Ile Ala Asn Asn Lys Lys Met

FIG. 8

FIGURE 9

FIGURE 10

Fig. 11

## DOCUMENTS CONSIDERED TO BE RELEVANT

EP 91103969.1

| Category | Citation of document with indication, where appropriate, of relevant passages | Relevant to claim | CLASSIFICATION OF THE APPLICATION (Int. Cl.5) |
|---|---|---|---|
| P,X | <u>WO - A1 - 90/07 341</u> (SYNERGEN) * Fig. 12; claims 4,11-15,25; page 1 * | 1, 3-15, 19-21 | C 12 N 15/12 C 07 H 21/04 C 12 N 1/21 C 12 N 5/10 C 12 N 5/16 C 07 K 13/00 A 61 K 37/02 |
| D,A | NATURE, vol. 342, No. 6252, December 21/28, 1989 (London) K.A. STÖCKLI et al. "Molecular cloning, expression and regional distribution of rat ciliary neurotrophic factor" pages 920-923 * Totality * | 1,11 | |
| D,A | SCIENCE, vol. 246, December 24, 1989 (Washington DC) L.-F. H. LIN et al. "Purification, cloning, and Expression of ciliary Neurotrophic Factor(CNTF)" pages 1023-1025 * Totality * | 1,11 | |

TECHNICAL FIELDS
SEARCHED (Int. Cl.5)

C 12 N
C 07 H
C 07 K
A 61 K

The present search report has been drawn up for all claims

| Place of search | Date of completion of the search | Examiner |
|---|---|---|
| VIENNA | 24-06-1991 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)